# EUROPÄISCHE PATENTSCHRIFT

(11) **EP 4 526 659 B1**
(45) Veröffentlichungstag und Bekanntmachung des Hinweises auf die Patenterteilung: **07.10.2026**
(21) Anmeldenummer: 23724237.5
(22) Anmeldetag: 02.05.2023
(51) Int. Cl.: G01N 21/41, G01N 21/15, G01N 33/28, G01N 21/84, G01N 21/85

(54) **EINRICHTUNG ZUR ERMITTLUNG DER MIT EINER WERKZEUGMASCHINE VERBUNDENEM TANK MIT WASSERMISCHBAREM KÜHLSCHMIERMITTEL ZUZUFÜHRENDEN MENGEN AN WASSER UND KONZENTRAT**
DEVICE FOR DETERMINING THE QUANTITIES OF WATER AND CONCENTRATE TO BE SUPPLIED TO A TANK CONTAINING A WATER-MISCIBLE COOLING LUBRICANT CONNECTED TO A MACHINE TOOL
DISPOSITIF PERMETTANT DE DÉTERMINER LES QUANTITÉS D'EAU ET DE CONCENTRÉ À ACHEMINER VERS LE RÉSERVOIR DE LUBRIFIANT RÉFRIGÉRANT MISCIBLE À L'EAU RELIÉ À UNE MACHINE-OUTIL

(30) Priorität: 14.05.2022 DE 102022001804
(43) Veröffentlichungstag der Anmeldung: 26.03.2025
(73) Patentinhaber: Leipoldt, Matthias, 08233 Treuen (DE)
(72) Erfinder: Leipoldt, Matthias, 08233 Treuen (DE)
(74) Vertreter: Kailuweit & Uhlemann Patentanwälte Partnerschaft mbB
(86) Internationale Anmeldenummer: PCT/EP2023/061455
(87) Internationale Veröffentlichungsnummer: WO 2023/222370

(56) Entgegenhaltungen:
- EP-A1- 3 964 834
- WO-A1-2020/225433
- WO-A1-2020/263964
- WO-A1-2021/001455
- DE-A1- 102010 028 319
- US-A- 3 838 646
- US-A1- 2020 209 032
- US-A1- 2020 292 519

## Beschreibung

Die Erfindung betrifft eine Einrichtung zur Ermittlung der mit einer Werkzeugmaschine verbundenem Tank mit wassermischbarem Kühlschmiermittel zuzuführenden Mengen an Wasser und Konzentrat.

Refraktometer zur Bestimmung des Brechungsindex sind allgemein bekannt. Dabei wird bekannterweise das Verhalten von Licht am Übergang zwischen einem Prisma mit bekannten Eigenschaften und dem zu prüfenden Stoff genutzt. Dabei wird ein Prisma als Messprisma eingesetzt, welches einen bekannten Brechungsindex aufweist. Das Licht breitet sich beim Übergang zwischen Prisma und hier wassermischbaren Kühlschmiermittel mit unterschiedlichen Winkeln aus, so dass der Brechungsindex des wassermischbaren Kühlschmiermittels messbar ist.

Die Druckschrift DE 10 2020 028 319 A1 offenbart ein Verfahren und eine Vorrichtung zur Steuerung der Konzentration wassergeschmierter Kühlschmierstoffe. Dazu sind in dem Kühlmittelkreislauf der Werkzeugmaschine ein Refraktometer zur Bestimmung der Brechzahl des wassergemischten Kühlschmierstoffs und ein Messgerät zur Messung der elektrischen Leitfähigkeit angeordnet. Das Refraktometer ist dazu als ein digitales Refraktometer ausgeführt, das als Lichtquelle eine LED und als Detektor einen CCD-Sensor aufweist.

Durch die Druckschrift DE 10 2019 206 613 A1 ist ein Inline-Refraktometer, insbesondere zur Ermittlung der Wasseranteile eines Kühlschmierstoffs bekannt. Das Inline-Refraktometer weist ein Prisma, ein Okular und eine Skala auf. Zur Ermittlung des Wasseranteils ist das Inline-Refraktometer einem Kreuzstück einer Rohrleitung zugeordnet. Dem Inline-Refraktometer ist weiterhin eine Kamera zum Ablesen des auf der Skala angezeigten Messwertes zugeordnet. Eine mengenmäßige Erfassung ist nicht vorgesehen.

Die Druckschrift US 3 838 646 A bezieht sich auf ein Geräuschunterdrückungssystem zur Verwendung in einem Rangierbahnhof für Eisenbahnen. Diese umfasst auch eine Mischeinrichtung zum Herstellen einer Öl-in-Wasser-Emulsion. Zur Analyse der Lösung im Mischtank wird ein nicht näher ausgeführtes Refraktometer verwendet.

Durch die Druckschrift EP 3 964 834 A1 sind ein Verfahren und eine Vorrichtung zur Überwachung einer Kühl-Schmierstoffemulsion bekannt. Dazu wird auch ein Refraktometer mit einem Messfenster verwendet. Ein Zulauf und ein Ablauf der Kühl-Schmierstoffemulsion sind winklig zum Messfenster angeordnet.

Durch die Druckschrift WO 2020/225433 A1 ist ein Inline-Refraktometer, insbesondere zur Ermittlung der Wasseranteile eines Kühlschmierstoffes, bekannt. Das Inline-Refraktometer weist ein Prisma, ein Okular und eine Skala auf. Zur Ermittlung des Wasseranteils ist das Inline-Refraktometer in einem Kreuzstück einer Rohrleitung eingebaut. Das Prisma des Inline-Refraktometers reicht in das Kreuzstück hinein. Weiterhin ist eine LED Lampe in das Kreuzstück integriert. Das Inline-Refraktometer bildet mit einer Kamera zum Ablesen des auf der Skala angezeigten Messwertes eine Refraktometereinheit. Das Kreuzstück ist Bestandteil einer Kühlschmierstoffleitung. Weiterhin ist ein Verfahren zum Dosieren des Wasseranteils oder des Ölanteils eines Gemisches in einem Leitungssystem Gegenstand der Druckschrift WO 2020/225433 A1. Mittels des Inline-Refraktometers wird der Brixwert des Gemisches im Leitungssystem gemessen. Bei Erreichen eines Schwellen-Brixwertes wird zusätzlich Kühlschmierstoff oder Wasser zugegeben.

Der im Patentanspruch 1 angegebenen Erfindung liegt die Aufgabe zugrunde, wassermischbares Kühlschmiermittel einfach automatisch zu überwachen.

Diese Aufgabe wird mit den im Patentanspruch 1 aufgeführten Merkmalen gelöst.

Die Einrichtung zur Überwachung von wassermischbaren Kühlschmiermittel zeichnet sich insbesondere durch eine automatische Überwachung des wassermischbaren Kühlschmiermittels aus.

Dazu weist eine Kammer einen ersten Anschluss zur bestimmten Zufuhr von Wasser, einen zweiten Anschluss zur bestimmten Zufuhr von wassermischbaren Konzentrat, einen dritten Anschluss zur Zufuhr von wassermischbaren Kühlschmiermittel der Werkzeugmaschine und einen vierten Anschluss zur Abfuhr des wassermischbaren Kühlschmiermittels aus der Kammer auf. Der erste Anschluss, der zweite Anschluss und der dritte Anschluss sind über einen Mischer mit dem Innenraum der Kammer verbunden. Der Innenraum der Kammer ist mit wenigstens einer Medium fördernden Vorrichtung verbunden. In der Kammer und so im wassermischbaren Kühlschmiermittel befindet sich ein Refraktometer. Das Refraktometer ist zwischen einer Quelle elektromagnetischer Strahlung und einer Digitalkamera oder einem Strahlungsdetektor angeordnet. Die Digitalkamera oder der Strahlungsdetektor ist mit einem Datenverarbeitungssystem verbunden, welches die Zufuhr von Wasser, die Zufuhr von wassermischbaren Konzentrat, die Zufuhr von wassermischbaren Kühlschmiermittel wenigstens einer Werkzeugmaschine und die Medium fördernde Vorrichtung so steuert, dass ein bestimmter Wert oder ein bestimmter Bereich der durch das wassermischbare Kühlschmiermittel hervorgerufenen Brechung erreicht und/oder eingehalten wird und die dabei bestimmten Mengen an Wasser, Konzentrat und wassermischbaren Kühlschmiermittel erfasst.

Wassermischbares Kühlschmiermittel dient bekannterweise beim Trennen und Umformen auf Werkzeugmaschinen der Wärmeabfuhr und der Schmierung. Hauptbestandteile sind Wasser und Öle. Dazu enthalten wassermischbare Konzentrate in Form von Emulgatoren und Mineralöl sowie Additive zur besseren Schmierung. Weiterhin sind mineralölfreie wassermischbare Kühlschmiermittel bekannt, welche beispielsweise Rapsöl enthalten können. Der Ölanteil der Emulsionen beträgt meist zwischen 5 und 8 Prozent. Wassermischbare Kühlschmiermittel müssen durch den Anwender regelmäßig überwacht werden. Der Einsatz von wassermischbaren Kühlschmiermittel verbessert die Standzeit der Werkzeuge und die Oberflächengüte der Werkstoffe. Deshalb ist insbesondere auch die Zusammensetzung bei der Verwendung des wassermischbaren Kühlschmiermittels wichtig. Um lange Verwendungszeiten zu erreichen, ist die Zusammensetzung des wassermischbaren Kühlschmiermittels weitestgehend gleichbleibend zu halten. Verbrauchte Bestandteile sind entsprechend dem Einsatz zu ergänzen. Dazu ist das wassermischbare Kühlschmiermittel zu prüfen, um die Menge zu ergänzender Bestandteile Wasser und/oder Konzentrat zu ermitteln. Mit der Überprüfung und einer bestimmten Ergänzung von Bestandteilen, kann die Verwendung des wassermischbaren Kühlschmiermittels verlängert werden. Damit sinken insbesondere die ansonsten hohen Entsorgungskosten. Gleichzeitig wird durch die längere Verwendung des wassermischbaren Kühlschmiermittels die Umwelt geschont. Mit der Einrichtung zur Überwachung von wassermischbaren Kühlschmiermittel können die zu ergänzenden Bestandteile einfach und automatisch ermittelt werden.

Dazu wird das Refraktometer verwendet, welches wenigstens ein Prisma besitzt. Das Refraktometer kann bekannterweise nach den Prinzipien Durchlicht, streifender Einfall oder Totalreflexion ausgebildet sein. Dabei wird ein Prisma mit einem bekannten Brechungsindex verwendet. Die elektromagnetische Strahlung in Form von Licht breitet sich beim Übergang zwischen Prisma und wassermischbaren Kühlschmiermittel mit unterschiedlichen Winkeln aus. Der unbekannte Brechungsindex des wassermischbaren Kühlschmiermittels wird über die Ablenkung der elektromagnetischen Strahlung gemessen. Vorteilhafterweise liegt wassermischbares Kühlschmiermittel in der Kammer direkt am Prisma an. Damit wird direkt der Brechungsindex des wassermischbaren Kühlschmiermittels bestimmt, wobei das Verhalten der elektromagnetischen Strahlung am Übergang zwischen dem Prisma mit bekannten Eigenschaften und dem wassermischbaren Kühlschmiermittel genutzt wird. Vorteilhafterweise kann wassermischbares Kühlschmiermittel vorbeiströmen, so dass durch Zugabe von Wasser und/oder Konzentrat die Zusammensetzung so veränderbar ist, dass wieder ein optimales wassermischbares Kühlschmiermittel zur Verfügung steht. Der Brechungsindex ist durch eine Kante und/oder einen Farbwechsel im Abbild gekennzeichnet, welches direkt mittels der Digitalkamera in digitale Daten zur Verarbeitung im Datenverarbeitungssystem gewandelt wird. Der Wert des Brechungsindex kann so im Verhältnis zur Abbildung und/oder einer Messskala ermittelt werden. Zur Kalibrierung kann so Wasser die untere Grenze und dem Konzentrat die obere Grenze zugeordnet werden. Die Messskala befindet sich dabei im Strahlengang der elektromagnetischen Strahlung zwischen Prisma und Digitalkamera. Das Abbild kann auch über den Strahlungsdetektor in digitale Daten umgesetzt werden. Dieser kann ein eindimensionaler Zeilensensor oder ein zweidimensionaler Arraysensor sein. Als Sensor kann so beispielsweise ein CCD-Sensor verwendet werden, wobei CCD für charge-coupled device steht.

Vorteilhafte Ausgestaltungen der Erfindung, gemäß Anspruch 1, sind in den folgenden Weiterbildungen und Ausführungsformen aufgeführt. Diese können die Einrichtung zur Überwachung von wassermischbaren Kühlschmiermittel einzeln oder in einer Kombination fortbilden, im Rahmen des durch Anspruch 1 definierten Umfangs.

Die Medium fördernde Vorrichtung ist erfindungsgemäß eine mit dem vierten Anschluss verbundene Pumpe zur Förderung von wassermischbaren Kühlschmiermittel aus dem Innenraum der Kammer. Weiterhin sind ein erstes Ventil mit dem ersten Anschluss, ein zweites Ventil mit dem zweiten Anschluss und ein drittes Ventil mit dem dritten Anschluss verbunden. Die Betätigungseinrichtungen des ersten Ventils, des zweiten Ventils und des dritten Ventils sind mit dem Datenverarbeitungssystem verbunden. Damit ist eine bestimmte Zuführung von Wasser, Konzentrat und/oder wassermischbaren Kühlschmiermittel gegeben. Vorteilhafterweise kann dazu eine Vakuumpumpe eingesetzt werden.

Erfindungsgemäß sind eine erste Wasser als Medium fördernde Vorrichtung (erstes Ventil) mit dem ersten Anschluss, eine zweite Konzentrat als Medium fördernde Vorrichtung (zweites Ventil) mit dem zweiten Anschluss und eine dritte wassermischbares Kühlschmiermittel als Medium fördernde Vorrichtung (drittes Ventil) mit dem dritten Anschluss verbunden. Diese Vorrichtungen (Ventile) und eine mit dem vierten Anschluss verbundene Pumpe sind mit dem

Datenverarbeitungssystem verbunden, welches die fördernden Vorrichtungen (Ventile und Pumpe) so steuert, dass
ein bestimmter Wert oder ein bestimmter Bereich der durch das wassermischbare Kühlschmiermittel hervorgerufenen Brechung erreicht und/oder eingehalten wird.

In Fortbildung können eine den Wasserdurchfluss messenden Vorrichtung, eine den Konzentratdurchfluss messenden Vorrichtung und eine den Kühlschmiermitteldurchfluss messenden Vorrichtung mit dem Datenverarbeitungssystem verbunden sein, welches die Wasser fördernde Vorrichtung, die Konzentrat fördernde Vorrichtung und die wassermischbares Kühlschmiermittel fördernde Vorrichtung so steuert, dass ein bestimmter Wert oder ein bestimmter Bereich der durch das wassermischbare Kühlschmiermittel hervorgerufenen Brechung erreicht und/oder eingehalten wird.

Vorteilhafterweise befindet sich wassermischbares Kühlschmiermittel der Kammer zwischen der Quelle elektromagnetischer Strahlung und einem Prisma des Refraktometers.

In einer Ausführungsform befindet sich das Refraktometer vorteilhafterweise zwischen den in einer Reihe beabstandet zueinander angeordneten Anschlüssen zur Zufuhr von Wasser, zur Zufuhr von Konzentrat und zur Zufuhr von wassermischbaren Kühlschmiermittel und dem vierten Anschluss zur Abfuhr von wassermischbarem Kühlschmiermittel. Damit kann wassermischbares Kühlschmiermittel am Prisma des Refraktometers vorbeiströmen.

Erfindungsgemäß ist der Mischer in einer Ausführungsform ein Rohr mit wenigstens einer im Rohr angeordneten Wendel. Diese ist schraubenförmig angeordnet. Vorteilhafterweise können auch zwei gegenläufig angeordnete Wendel im Rohr angeordnet sein.

An der Kammer befindet sich erfindungsgemäß ein Ultraschallschwinger, so dass Ultraschall in das wassermischbare Kühlschmiermittel im Innenraum der Kammer eingeleitet wird. Dieser führt vorteilhafterweise dazu, dass eventuell vorhandene Ablagerungen von der Oberfläche des Prismas gelöst werden. Dazu kann der Ultraschallschwinger insbesondere auch parallel zum Prisma des Refraktometers angeordnet sein.

Das Refraktometer kann in einer Ausführungsform eine Reinigungsvorrichtung aufweisen. Das kann beispielsweise ein Wischer sein.

Mit der Reinigung des Refraktometers kann die Einrichtung zur Überwachung von wassermischbaren Kühlschmiermittel bis auf kurzzeitige Reinigungen des Refraktometers kontinuierlich betrieben werden.

Die Einrichtung zur Überwachung von wassermischbaren Kühlschmiermittel kann in einer Ausführungsform ein Luxmeter zur Messung der Beleuchtungsstärke der Quelle elektromagnetischer Strahlung aufweisen, welches mit dem Datenverarbeitungssystem verbunden ist. In Verbindung mit dem Abbild ist so die Funktion der Einrichtung kontrollierbar. Das Refraktometer befindet sich in einer Ausführungsform zwischen der Quelle elektromagnetischer Strahlung und einer vor der Digitalkamera angeordneten Messskala Das Datenverarbeitungssystem ist dabei so ausgebildet, dass das Abbild der Messskala und der Brechungsindex aus der Brechung der elektromagnetischen Strahlung mit einem Datensichtgerät dargestellt wird.

Das Datenverarbeitungssystem ist erfindungsgemäß so ausgebildet, dass aus den ermittelten Mengen an Wasser, Konzentrat und wassermischbares Kühlschmiermittel die einem mit einer Werkzeugmaschine verbundenem Tank mit wassermischbarem Kühlschmiermittel zuzuführenden Mengen an Wasser und Konzentrat ermittelt werden.

Ein Ausführungsbeispiel der Erfindung ist in den Zeichnungen jeweils prinzipiell dargestellt und wird im Folgenden näher beschrieben.

Es zeigen:
- Fig. 1: eine Einrichtung zur Überwachung von wassermischbaren Kühlschmiermittel,
- Fig. 2: eine Kammer mit einem Ultraschallschwinger und
- Fig. 3: ein Blockschaltbild der Einrichtung zur Überwachung von wassermischbaren Kühlschmiermittel.

Eine Einrichtung zur Überwachung von wassermischbaren Kühlschmiermittel besteht im Wesentlichen aus einer Kammer 1 mit einem ersten Anschluss 2, einem zweiten Anschluss 3, einem dritten Anschluss 4, einem vierten Anschluss 5, einem Mischer 6 einem Refraktometer 7, einer Quelle elektromagnetischer Strahlung 8 und einer Digitalkamera 9.

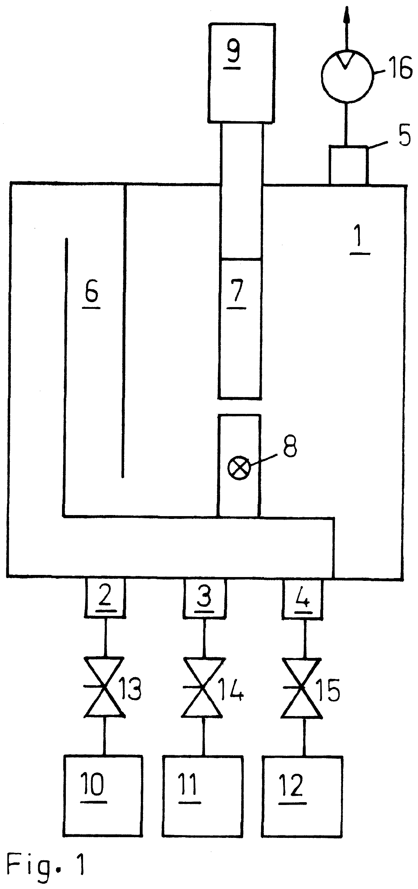

Die Fig. 1 zeigt eine Einrichtung zur Überwachung von wassermischbaren Kühlschmiermittel in einer prinzipiellen Darstellung.

Die Kammer 1 besitzt den ersten Anschluss 2, den zweiten Anschluss 3 und den dritten Anschluss 4, wobei
- der erste Anschluss 2 zur bestimmten Zufuhr von Wasser 10 über ein erstes Ventil 13 mit einem Reservoir mit Wasser 10,
- der zweite Anschluss 3 zur bestimmten Zufuhr von wassermischbaren Konzentrat 11 über ein zweites Ventil 14 mit einem Reservoir mit Konzentrat 11 und
- der dritte Anschluss 4 zur Zufuhr von wassermischbaren Kühlschmiermittel 4 mit einem Reservoir mit wassermischbarem Kühlschmiermittel 12 wenigstens einer Werkzeugmaschine verbunden sind.

Der vierte Anschluss 5 zur Abfuhr des wassermischbaren Kühlschmiermittels 12 aus der Kammer 1 ist mit einer Medium fördernden Vorrichtung 16 beispielsweise in Form einer Vakuumpumpe 16 verbunden.

Der erste Anschluss 2, der zweite Anschluss 3 und der dritte Anschluss 4 sind über den Mischer 6 mit dem Innenraum der Kammer 1 verbunden. Der Mischer 6 ist ein Rohr 6 mit wenigstens einer im Rohr 6 angeordneten Wendel, welche dazu schraubenförmig ausgebildet ist. Diese kann einläufig oder ineinander mehrläufig ausgebildet sein. Weiterhin kann das Rohr 6 als Mischer 6 auch mäanderförmig angeordnet sein.

In der Kammer 1 und so im wassermischbaren Kühlschmiermittel 12 befindet sich das Refraktometer 7, wobei dieses zwischen der Quelle 8 elektromagnetischer Strahlung und der Digitalkamera 9 so angeordnet ist, dass das Abbild aus der Brechung der elektromagnetischen Strahlung mittels der Digitalkamera 9 digitalisiert wird. Dazu befindet sich wassermischbares Kühlschmiermittel 12 der Kammer 1 zwischen der Quelle 8 elektromagnetischer Strahlung und einem Prisma des Refraktometers 7. Der erste Anschluss 2, der zweite Anschluss 3 und der dritte Anschluss 4 sind in einer Reihe angeordnet, so dass sich das Refraktometer 7 zwischen den in einer Reihe beabstandet zueinander angeordneten Anschlüssen 2, 3, 4 zur Zufuhr von Wasser 10, zur Zufuhr von Konzentrat 11 und zur Zufuhr von wassermischbaren Kühlschmiermittel 12 und dem vierten Anschluss 5 zur Abfuhr von wassermischbaren Kühlschmiermittel 12 befindet.

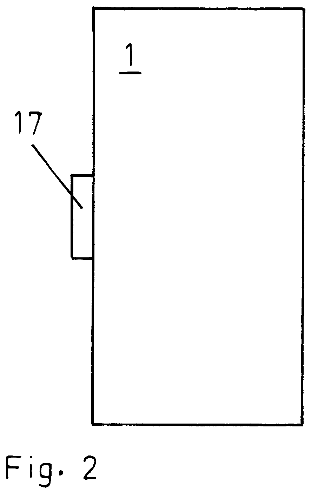

Die Fig. 2 zeigt eine Kammer 1 mit einem Ultraschallschwinger 17 in einer prinzipiellen Darstellung.

An der Kammer 1 ist wenigstens ein Ultraschallschwinger 17 so angebracht, dass der Ultraschall in das wassermischbare Kühlschmiermittel 12 im Innenraum der Kammer 1 eingeleitet wird, so dass eventuell vorhandene Ablagerungen von der Oberfläche des Prismas des Refraktometers gelöst werden. Dazu kann der Ultraschallschwinger 17 insbesondere parallel zum Prisma des Refraktometers 7 angeordnet sein.

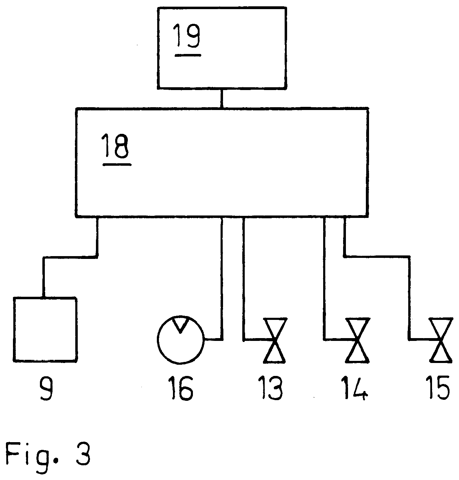

Die Fig. 3 zeigt ein Blockschaltbild der Einrichtung zur Überwachung von wassermischbaren Kühlschmiermittel 12 in einer prinzipiellen Darstellung.

Die Betätigungseinrichtungen des ersten Ventils 13, des zweiten Ventils 14, des dritten Ventils 15, die wassermischbares Kühlschmiermittel 12 als Medium fördernde Vorrichtung 16 und die Digitalkamera 9 sind mit einem Datenverarbeitungssystem 18 verbunden, welches die Zufuhr von Wasser 10, die Zufuhr von wassermischbaren Konzentrat 11, die Zufuhr von wassermischbaren Kühlschmiermittel 12 der Werkzeugmaschine und die Medium fördernde Vorrichtung 16 so steuert, dass ein bestimmter Wert oder ein bestimmter Bereich der durch das wassermischbare Kühlschmiermittel 12 hervorgerufenen Brechung erreicht und/oder eingehalten wird und die dabei bestimmten Mengen an Wasser 10, Konzentrat 11 und wassermischbaren Kühlschmiermittel 12 erfasst. Dazu kann das Datenverarbeitungssystem 18 mit einem Bildschirm 19 verbunden sein.

In einer Ausführungsform kann ein Luxmeter zur Messung der Beleuchtungsstärke der Quelle 8 elektromagnetischer Strahlung vorhanden sein, welches mit dem Datenverarbeitungssystem 18 verbunden ist.

Darüber hinaus kann sich das Refraktometer 7 zwischen der Quelle 8 elektromagnetischer Strahlung und einer vor der Digitalkamera 9 angeordneten Messskala befinden. Das Datenverarbeitungssystem 18 kann dazu so ausgebildet sein, dass das Abbild der Messskala und der Brechungsindex aus der Brechung der elektromagnetischen Strahlung mit dem Bildschirm 19 als Datensichtgerät dargestellt wird.

Weiterhin ist das Datenverarbeitungssystem 18 so ausgebildet, dass aus den ermittelten Mengen an Wasser 10, Konzentrat 11 und wassermischbaren Kühlschmiermittel 12 die einem mit einer Werkzeugmaschine verbundenem Tank mit wassermischbarem Kühlschmiermittel 12 zuzuführenden Mengen an Wasser 10 und Konzentrat 11 ermittelt werden.

### Bezugszeichen

- 1: Kammer
- 2: erster Anschluss
- 3: zweiter Anschluss
- 4: dritter Anschluss
- 5: vierter Anschluss
- 6: Rohr / Mischer
- 7: Refraktometer
- 8: elektromagnetische Strahlung
- 9: Digitalkamera
- 10: Reservoir mit Wasser
- 11: wassermischbares Konzentrat
- 12: wassermischbares Kühlschmiermittel
- 13: erstes Ventil
- 14: zweites Ventil
- 15: drittes Ventil
- 16: Medium fördernden Vorrichtung
- 17: Ultraschallschwinger
- 18: Datenverarbeitungssystem
- 19: Datensichtgerät, z. B. Bildschirm

## Patentansprüche

1. Einrichtung zur Ermittlung der mit einer Werkzeugmaschine verbundenem Tank mit wassermischbarem Kühlschmiermittel (12) zuzuführenden Mengen an Wasser (10) und Konzentrat (11) umfassend
- eine Kammer (1) mit einem ersten Anschluss (2) zur bestimmten Zufuhr von Wasser (10), einem zweiten Anschluss (3) zur bestimmten Zufuhr von wassermischbarem Konzentrat (11), einem dritten Anschluss (4) zur Zufuhr von wassermischbarem Kühlschmiermittel (12) wenigstens einer Werkzeugmaschine und einem vierten Anschluss (5) zur Abfuhr des wassermischbaren Kühlschmiermittels (12) aus der Kammer (1),
- eine mit dem vierten Anschluss (5) verbundene Pumpe (16) zur Förderung von wassermischbarem Kühlschmiermittel (12) aus dem Innenraum der Kammer (1),
- ein mit dem ersten Anschluss (2) verbundenes erstes Ventil (13),
- ein mit dem zweiten Anschluss (3) verbundenes zweites Ventil (14),
- ein mit dem dritten Anschluss (4) verbundenes drittes Ventil (15),
- einen den ersten Anschluss (2), den zweiten Anschluss (3) und den dritten Anschluss (4) mit dem Innenraum der Kammer (1) verbindenden Mischer (6), wobei der Mischer (6) ein Rohr mit wenigstens einer im Rohr schraubenförmig angeordneten Wendel ist,
- ein Prisma eines Refraktometers (7), so dass wassermischbares Kühlschmiermittel in der Kammer (1) direkt am Prisma anliegt,
- eine Quelle (8) elektromagnetischer Strahlung und eine Digitalkamera (9), so dass das Prisma des Refraktometers (7) zwischen der Quelle (8) elektromagnetischer Strahlung und der Digitalkamera (9) angeordnet ist,
- ein mit den Betätigungseinrichtungen der Ventile (13, 14, 15), der Pumpe (16) und der Digitalkamera (9) verbundenes Datenverarbeitungssystem (18), welches die Zufuhr von Wasser (10), die Zufuhr von wassermischbarem Konzentrat (11), die Zufuhr von wassermischbarem Kühlschmiermittel (12) der Werkzeugmaschine und die Pumpe (16) so steuert, dass ein bestimmter Wert oder ein bestimmter Bereich der durch das wassermischbare Kühlschmiermittel (12) hervorgerufenen Brechung erreicht und/oder eingehalten wird und die dabei bestimmten Mengen an Wasser (10), Konzentrat (11) und wassermischbarem Kühlschmiermittel (12) erfasst,
und
- wenigstens einen an der Kammer (1) angebrachten Ultraschallschwinger (17), so dass der Ultraschall in das wassermischbare Kühlschmiermittel (12) im Innenraum der Kammer (1) eingeleitet wird, so dass eventuell vorhandene Ablagerungen von der Oberfläche des Prismas des Refraktometers (7) gelöst werden.

2. Einrichtung nach Patentanspruch 1, **dadurch gekennzeichnet, dass** sich wassermischbares Kühlschmiermittel (12) der Kammer zwischen der Quelle (8) elektromagnetischer Strahlung und dem Prisma des Refraktometers (7) befindet.

3. Einrichtung nach Patentanspruch 1, **dadurch gekennzeichnet, dass** ein Luxmeter zur Messung der Beleuchtungsstärke der Quelle (8) elektromagnetischer Strahlung vorhanden ist und dass das Luxmeter mit dem Datenverarbeitungssystem (18) verbunden ist.

4. Einrichtung nach Patentanspruch 1, **dadurch gekennzeichnet, dass** sich das Refraktometer (7) zwischen der Quelle (8) elektromagnetischer Strahlung und einer vor der Digitalkamera (9) angeordneten Messskala befindet und dass das Datenverarbeitungssystem (18) so ausgebildet ist, dass das Abbild der Messskala und der Brechungsindex aus der Brechung der elektromagnetischen Strahlung mit einem Datensichtgerät (19) dargestellt wird.

## Claims

1. Device for determining the quantities of water (10) and concentrate (11) to be supplied to a tank containing water-miscible cooling lubricant (12) connected to a machine tool, comprising
- a chamber (1) with a first port (2) for the specific supply of water (10), a second port (3) for the specific supply of water-miscible concentrate (11), a third port (4) for the supply of water-miscible cooling lubricant (12) to at least one machine tool and a fourth port (5) for discharge of the water-miscible cooling lubricant (12) from the chamber (1),
- a pump (16) connected to the fourth port (5) for the transport of water-miscible cooling lubricant (12) from the interior of the chamber (1),
- a first valve (13) connected to the first port (2),
- a second valve (14) connected to the second port (3),
- a third valve (15) connected to the third port (4),
- a mixer (6) connecting the first port (2), the second port (3) and the third port (4) to the interior of the chamber (1), wherein the mixer (6) is a tube with at least one coil arranged helically in the tube,
- a prism of a refractometer (7) such that water-miscible cooling lubricant in the chamber (1) is directly adjacent to the prism,
- a source (8) of electromagnetic radiation and a digital camera (9), such that the prism of the refractometer (7) is arranged between the source (8) of electromagnetic radiation and the digital camera (9),
- a data processing system (18) connected to the actuating devices of the valves (13, 14, 15), the pump (16) and the digital camera (9), which controls the supply of water (10), the supply of water-miscible concentrate (11), the supply of water-miscible cooling lubricant (12) to the machine tool and the pump (16) in such a way that a certain value or a certain range of the refraction caused by the water-miscible cooling lubricant (12) is reached and/or maintained and records the quantities of water (10), concentrate (11) and water-miscible cooling lubricant (12) determined thereby,
and
- at least one ultrasonic transducer (17) attached to the chamber (1) so that the ultrasound is introduced into the water-miscible cooling lubricant (12) in the interior of the chamber (1) so that any deposits that may be present are removed from the surface of the prism of the refractometer (7).

2. Device according to claim 1, **characterized in that** water-miscible cooling lubricant (12) is located in the chamber between the source (8) of electromagnetic radiation and the prism of the refractometer (7).

3. Device according to claim 1, **characterized in that** a lux meter for the measurement of the illuminance of the source (8) of electromagnetic radiation is present and that the lux meter is connected to the data processing system (18) is connected.

4. Device according to claim 1, **characterized in that** the refractometer (7) is located between the source (8) of electromagnetic radiation and a measuring scale arranged in front of the digital camera (9) and that the data processing system (18) is designed such that the image of the measuring scale and the refractive index from the refraction of electromagnetic radiation displayed with a data display device (19).

## Revendications

1. Dispositif de détermination des quantités d'eau (10) et de concentré (11) à fournir à un réservoir contenant du lubrifiant de refroidissement miscible à l'eau (12) relié à une machine-outil, comprenant
- une chambre (1) avec un premier orifice (2) pour l'alimentation spécifique en eau (10), un deuxième orifice (3) pour l'alimentation spécifique en concentré miscible à l'eau (11), un troisième orifice (4) pour l'alimentation en lubrifiant de refroidissement miscible à l'eau (12) à au moins une machine-outil et un quatrième orifice (5) pour l'évacuation du lubrifiant de refroidissement miscible à l'eau (12) de la chambre (1),
- une pompe (16) reliée au quatrième orifice (5) pour le transport de lubrifiant de refroidissement miscible à l'eau (12) depuis l'intérieur de la chambre (1),
- une première vanne (13) reliée au premier orifice (2),
- une deuxième vanne (14) reliée au deuxième orifice (3),
- une troisième vanne (15) reliée au troisième orifice (4),
- un mélangeur (6) reliant le premier orifice (2), le deuxième orifice (3) et le troisième orifice (4) à l'intérieur de la chambre (1), dans lequel le mélangeur (6) est un tube comportant au moins une hélice disposée hélicoïdalement dans le tube,
- un prisme d'un réfractomètre (7) tel que du lubrifiant de refroidissement miscible à l'eau dans la chambre (1) soit directement adjacent au prisme,
- une source (8) de rayonnement électromagnétique et un appareil photo numérique (9), de sorte que le prisme du réfractomètre (7) soit disposé entre la source (8) de rayonnement électromagnétique et l'appareil photo numérique (9),
- un système de traitement de données (18) connecté aux dispositifs d'actionnement des vannes (13, 14, 15), à la pompe (16) et à l'appareil photo numérique (9), qui contrôle l'alimentation en eau (10), l'alimentation en concentré miscible à l'eau (11), l'alimentation en lubrifiant de refroidissement miscible à l'eau (12) à la machine-outil et la pompe (16) de manière à atteindre et/ou maintenir une certaine valeur ou une certaine plage de réfraction causée par le lubrifiant de refroidissement miscible à l'eau (12), et enregistre les quantités d'eau (10), de concentré (11) et de lubrifiant de refroidissement miscible à l'eau (12) déterminées dans ce processus.
et
- au moins un transducteur ultrasonique (17) fixé à la chambre (1) afin que les ultrasons soient introduits dans le lubrifiant de refroidissement miscible à l'eau (12) à l'intérieur de la chambre (1) afin que des dépôts éventuellement présents soient éliminés de la surface du prisme du réfractomètre (7).

2. Dispositif selon la revendication 1, **caractérisé en ce que** le lubrifiant de refroidissement miscible à l'eau (12) est situé dans la chambre entre la source (8) de rayonnement électromagnétique et le prisme du réfractomètre (7).

3. Dispositif selon la revendication 1, **caractérisé en ce qu'**un luxmètre est prévu pour mesurer l'éclairement de la source (8) de rayonnement électromagnétique et que le luxmètre est connecté au système de traitement de données (18).

4. Dispositif selon la revendication 1, **caractérisé en ce que** le réfractomètre (7) est situé entre la source (8) de rayonnement électromagnétique et une échelle de mesure disposée devant l'appareil photo numérique (9) et que le système de traitement des données (18) est conçu de telle sorte que l'image de l'échelle de mesure et l'indice de réfraction de la réfraction du rayonnement électromagnétique soit affiché avec un dispositif d'affichage de données (19).
